(19) Europäisches Patentamt
European Patent Office
Office européen des brevets

(11) Veröffentlichungsnummer: **0 235 804**
**B1**

(12) # EUROPÄISCHE PATENTSCHRIFT

(45) Veröffentlichungstag der Patentschrift: **06.12.89**

(51) Int. Cl.⁴: **C07C 5/367,** C07C 15/06, C07C 15/085, C07C 5/41

(21) Anmeldenummer: **87103020.1**

(22) Anmeldetag: **04.03.87**

(54) **Verbessertes Verfahren zur Herstellung von p-Cymol und homologen Alkylbenzolen.**

(30) Priorität: **07.03.86 DE 3607448**

(43) Veröffentlichungstag der Anmeldung: **09.09.87 Patentblatt 87/37**

(45) Bekanntmachung des Hinweises auf die Patenterteilung: **06.12.89 Patentblatt 89/49**

(84) Benannte Vertragsstaaten: **CH DE ES FR GB IT LI NL**

(56) Entgegenhaltungen:
**EP-A- 0 022 297**
**DE-A- 2 905 806**
**US-A- 4 087 476**

(73) Patentinhaber: **BASF Aktiengesellschaft, Carl-Bosch-Strasse 38, D-6700 Ludwigshafen(DE)**

(72) Erfinder: **Martin, Roland, Dr., Bruesseler Ring 53, D-6700 Ludwigshafen(DE)**
Erfinder: **Gramlich, Walter, Dr., Auf der Hoehe 11, D-6803 Edingen-Neckarhausen(DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die vorliegende Erfindung betrifft ein Verfahren zur Herstellung von Alkylbenzolen, insbesondere von p-Cymol, durch katalytische Umsetzung der entsprechenden Alkenylcyclohexene.

Aus US 3 312 635 ist die Umsetzung von Limonen an Nickel- und Molybdänoxid enthaltenden Trägerkatalysatoren bei Temperaturen zwischen 200 und 250°C zu Gemischen aus p-Cymol, p-Menthan und p-Menthen bekannt.

Aus Bull. Chem. Soc. Japan 1978, Band 51, S. 3641-42, ist bekannt, daß die oben angeführte Umsetzung in Gegenwart von Wasserstoff auch an Magnesium-, Calcium- oder Lanthanoxid durchgeführt werden kann.

Weiterhin ist aus US 2 857 439 bekannt, daß man an zahlreichen Katalysatoren, insbesondere an Palladium als Katalysator, hauptsächlich p-Cymol erhält, wenn man monocyclische Terpene in Dampfform über die erhitzten Katalysatoren leitet und die Dämpfe anschließend kondensiert. In dieser Patentschrift heißt es, daß diese bekannten Verfahren bei Verwendung von mit Schwefel verunreinigten Terpenen nicht vorteilhaft seien. Daher wurde in loc.cit. für mit Schwefel verunreinigte Terpene das Überleiten ihrer Dämpfe in Gegenwart von Wasserstoff über besondere, auf 200 bis 500°C erhitzte Platin-Trägerkatalysatoren beschrieben.

Aus US 2 402 898 ist außerdem bekannt, daß monocyclische Terpene an Katalysatoren, die Edelmetalle wie Pd und Pt auf Aktivkohle enthalten, bei ca. 300°C zu p-Cymol aromatisiert werden können. Nachteilig an diesem Verfahren ist, daß der Katalysator nur relativ kurze Zeit eine ausreichende Aktivität behält.

Neuerdings wurde außerdem durch das europäische Patent 77 289 ein Verfahren zur Umwandlung von Terpenen in Cymole durch Erhitzen dieser Terpene in Gegenwart eines Alkalimetallcarbonat-Katalysators auf einem MgO-Träger, der eine Oberfläche von wenigstens 20 $m^2/g$ besitzt, bekannt. Gemäß den Beispielen dieses Patents werden Terpentinöl, Limonen und Δ-3-Caren umgesetzt. Nachteilig in diesem Verfahren ist, daß man Reaktionstemperaturen von 400°C und mehr benötigt, was einerseits einen sehr hohen Energieaufwand bedeutet und andererseits eine relativ schnelle Desaktivierung des Katalysators durch Zersetzungsprodukte erwarten läßt.

Der Erfindung lag nun die Aufgabe zugrunde, einen Katalysator zur Ver fügung zu stellen, der es ermöglicht, Alkenylcyclohexene der allgemeinen Formel II besonders vorteilhaft in die entsprechende Alkylbenzole zu überführen. Der Katalysator sollte sich durch einfache Verfügbarkeit, lange Standzeiten, hohe Aktivität und leichte Regenerierbarkeit auszeichnen und möglichst hohe Umsätze mit guter Selektivität bei möglichst geringen Reaktionstemperaturen gewährleisten.

Gegenstand der Erfindung ist nun ein Verfahren zur Herstellung von Alkylbenzolen der allgemeinen Formel I

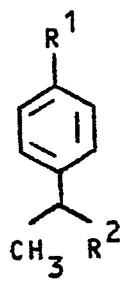

(I),

in der $R^1$ für einen $C_1$- bis $C_4$-Alkylrest, vorzugsweise für den Methylrest steht und $R^2$ Wasserstoff oder einen $C_1$-$C_4$-Alkylrest, vorzugsweise eine Methylgruppe bedeutet, durch katalytische Aromatisierung von Alkenylcyclohexenen der allgemeinen Formel II

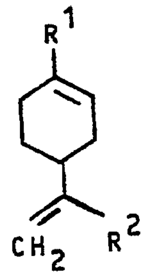

(II),

in der $R^1$ und $R^2$ die oben angegebene Bedeutung haben, das dadurch gekennzeichnet ist, daß man die Umsetzung in der Gasphase bei 150 bis 350°C an einem Katalysator durchführt, der Palladiumoxid und Schwefel und/oder Selen bzw. Selenoxid auf Aktivkohle enthält.

Im erfindungsgemäßen Verfahren wurden die eingangs an den Katalysator gestellten Anforderungen weitgehend erfüllt. Überraschenderweise gelang die Aromatisierung der Alkenylcyclohexene der Formel I an den erfindungsgemäßen Katalysatoren in sehr hohen Ausbeuten und mit nur sehr geringer Nebenproduktbildung. Die Dehydrierung kann in Abwesenheit von Luft oder Sauerstoff durchgeführt werden, wodurch ein wesentlicher Beitrag zur Sicherheit der technischen Durchführung geleistet wird. Überraschend war auch die lange Lebensdauer des Katalysators von bis zu mehreren Monaten gegenüber beispielsweise nur 50 bis 100 Stunden bei dem gemäß dem Verfahren von US 2 402 898 benutzten Katalysator.

Dies ist besonders überraschend, da in US 2 857 439 sehr überzeugend dargelegt wird, daß bei Vorhandensein von Schwefel in den als Ausgangsstoff verwendeten Terpenen mit den bekannten Verfahren zur Aromatisierung von Terpenen nur sehr unbefriedigende Ergebnisse erzielt würden, da der Schwefel die verwendeten Katalysatoren verunreinigt und dadurch vergiftet, was zu extrem kurzen Katalysatorstandzeiten führt.

Nach dem Abfallen der Aktivität kann der erfindungsgemäße Katalysator auf einfache Weise durch Luftoxidation wieder regeneriert werden.

Als Katalysatoren werden für das erfindungsgemäße Verfahren Aktivkohlen verwendet, die 0,5 bis 10 Gew.%, vorzugsweise 1 bis 7 Gew.% Palladiumoxid und 0,1 bis 2, vorzugsweise 0,2 bis 1 Gew.% an Schwefel und/oder Selen bzw. Selendioxid enthalten.

Im allgemeinen werden die Katalysatoren in Form von Strängen von 2 bis 5 mm Länge oder Tabletten mit 1 bis 4 mm Durchmesser eingesetzt.

Die Form des Katalysators richtet sich nach dem Reaktionsgefäß. Für den Fall der Umsetzung in einem Wirbelbettreaktor verwendet man einen pulverförmigen Wirbelkontakt mit Teilchengrößen von 0,1 bis 0,3 mm.

Zur Herstellung des erfindungsgemäßen Katalysators geht man im allgemeinen so vor, daß man die Aktivkohle bzw. die Aktivkohlepresslinge in einer Imprägniertrommel zunächst in an sich bekannter Weise mit einer Palladiumnitratlösung besprüht, das Produkt bei erhöhter Temperatur gut trocknet und dann in einem zweiten Imprägnierschritt eine Ammoniumpolysulfidlösung bzw. eine wäßrige Lösung von seleniger Säure aufsprüht und schließlich wieder bei erhöhter Temperatur gut trocknet.

Die erfindungsgemäße Umsetzung wird vorzugsweise kontinuierlich bei Normaldruck nach den dafür üblichen Techniken, wie in einem Strömungsreaktor oder einem Wirbelbettreaktor durchgeführt.

Die Aromatisierung wird bei Temperaturen von 150 bis 350°C, vorzugsweise 200 bis 300°C durchgeführt und zwar in der Weise, daß das Alkenylcyclohexen verdampft und mit Stickstoff als Trägergas über den heißen Katalysator geleitet wird. Das Verhältnis von Alkenylcyclohexendampf zu Stickstoff beträgt dabei im allgemeinen 1:1 bis 1:5, vorzugsweise 1,5 bis 2,5. Die Aufarbeitung erfolgt auf übliche Weise, z.B. durch Destillation.

Die als Ausgangsverbindungen benötigten Alkenylcyclohexene sind in vielen Pflanzen enthalten oder fallen bei verschiedenen industriellen Prozessen als Nebenprodukte an. So ist beispielsweise Limonen in Südfrüchten enthalten und fällt bei der Herstellung von Fruchtsäften aus Orangen und/oder Citronen in großer Menge als Nebenprodukt an.

Das eingesetzte Alkenylcyclohexen muß nicht unbedingt 100 %ig rein sein, doch ist ein Gehalt von mehr als 90 % zu empfehlen, wenn das entstehende Alkylbenzol ohne weitere Reinigung verwendet werden soll. Dies gilt besonders für die Aromatisierung von Limonen, da das daraus entstehende p-Cymol selbst als Riechstoff Verwendung findet und außerdem als Vorprodukt für weitere begehrte Riechstoffe wie Cuminaldehyd und Cyclamenaldehyd eingesetzt wird, wofür eine hohe Reinheit des p-Cymols Voraussetzung ist.

Die nachfolgenden Beispiele veranschaulichen den Gegenstand der Erfindung.

<u>Beispiele 1 bis 3</u>

<u>Vergleichsbeispiele 1 und 2</u>

Jeweils die aus der folgenden Tabelle ersichtliche Menge an d-Limonen (Reinheit: 96 GC FL%) wurde verdampft und mit Stickstoff im Verhältnis von etwa 1:2 verdünnt über den in einem Rohrreaktor (600 mm lang und 30 mm im Durchmesser) befindlichen Katalysator geleitet, der auf die aus der Tabelle ersichtliche Temperatur aufgeheizt wurde, der die aus der Tabelle ersichtliche Zusammensetzung aufwies und in Form von 4 mm-Strängen vorlag. Die Bestimmung der Ausgangs- und Reaktionsprodukte erfolgte gaschromatographisch sowie durch $^1$H-NMR- und IR-spektroskopische Untersuchungen. Der Umsatz betrug in allen Versuchen 100 %.

Tabelle

| Beispiel | Katalysator Zusammensetzung | Menge [g] | Temperatur [°C] | d-Limonen [kg] | erhaltenes p-Cymol [kg] | Reinheit [GC FL%] | Ausbeute [% d. Theorie] |
|---|---|---|---|---|---|---|---|
| 1 | 5,75% PdO + 0,25% S auf Aktivkohle | 90 | 220–230 | 62 | 59,5 | 96 | 97 |
| 2 | 5,75% PdO + 1% S auf Aktivkohle | 86 | 250 | 31,5 | 31 | 95 | 98 |
| 3 | 5,75% PdO + Se (ber. 0,35% $SeO_2$) auf Aktivkohle | 98 | 260 | 9,06*) | 8,6 | 95 | 92 |
| Vergleichsbeispiel | | | | | | | |
| 1 | 0,5% Pd auf $Al_2O_3$ | 100 | 230 | 1,89 | 1,8 | 70 | 69 |
| 2 | 5% Pd auf Aktivkohle | 118 | 230 | 5,03 | 4,7 | 89 | 88 |

*) Reinheit 99%

Beispiel 4

Jeweils etwa 1 l/h (5,8 mol/h) an α-Limonen (Reinheit: 99 GC Fl%) wurde verdampft, was etwa 130 l/h Limonendampf ergab. Dieser wurde mit etwa 200 bis 250 l $N_2$/h verdünnt über einen in einem Rohrreaktor (2 m lang; 4 cm Durchmesser; 3 l Inhalt) befindlichen auf 230 bis 250°C aufgeheizten Katalysator der Zusammensetzung 5,75 % PdO + 0,25 % S auf Aktivkohlepresslingen (4-mm-Stränge) geleitet. Auch nach Umsetzen von 1490 kg Limonen am gleichen Katalysator im Verlauf von etwa 3 Monaten war der Katalysator noch genau so aktiv wie am Anfang des Langzeitversuchs; der Umsatz betrug immer 100 %; die Ausbeute 96 bis 97 % der Theorie.

**Patentansprüche**

1. Verfahren zur Herstellung von Alkylbenzolen der allgemeinen Formel I

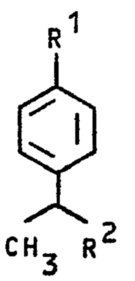

(I),

in der $R^1$ für einen $C_1$- bis $C_4$-Alkylrest steht und $R^2$ Wasserstoff oder einen $C_1$-$C_4$-Alkylrest bedeutet, durch katalytische Aromatisierung von Alkenylcyclohexenen der allgemeinen Formel II

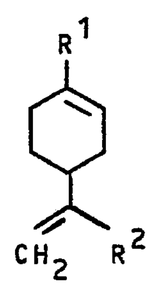

(II),

in der $R^1$ und $R^2$ die oben angegebene Bedeutung haben, dadurch gekennzeichnet, daß man die Umsetzung in der Gasphase bei 150 bis 350°C an einem Katalysator durchführt, der Palladiumoxid und Schwefel und/oder Selen bzw. Selenoxid auf Aktivkohle enthält.

2. Verfahren gemäß Anspruch 1, dadurch gekennzeichnet, daß man die Umsetzung unter Ausschluß von Luft oder Sauerstoff durchführt.

3. Verfahren gemäß Anspruch 1, dadurch gekennzeichnet, daß man die Umsetzung bei 2OO bis 3OO°C durchführt.

4. Verfahren gemäß Anspruch 1, dadurch gekennzeichnet, daß man die Umsetzung an einem Katalysator durchführt, der Palladiumoxid in einer Menge von O,5 bis 1O Gew.% sowie O,1 bis 2 Gew.% an Schwefel und/oder Selen bzw. Selenoxid enthält.

5. Verfahren gemäß Anspruch 1, dadurch gekennzeichnet, daß man zur Herstellung von p-Cymol Limonen verdampft und mit Stickstoff als Trägergas über den heißen Katalysator leitet.

**Revendications**

1. Procédé de préparation d'alkylbenzènes de formule générale I

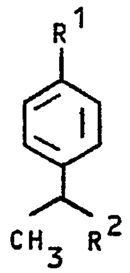

(I),

dans laquelle $R^1$ représente un groupe alkyle en $C_1$–$C_4$ et
$R^2$ représente l'hydrogène ou un groupe alkyle en $C_1$–$C_4$, par aromatisation catalytique d'alcénylcyclohexènes de formule générale II

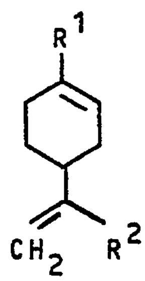

(II),

dans laquelle $R^1$ et $R^2$ ont les significations indiquées ci-dessus, caractérisé par le fait que l'on effectue la réaction en phase gazeuse à des températures de 150 à 350°C sur un catalyseur contenant de l'oxyde de palladium et du soufre et/ou du sélénium ou respectivement de l'oxyde de sélénium sur charbon actif.

2. Procédé selon la revendication 1, caractérisé par le fait que l'on effectue la réaction à l'abri de l'air ou de l'oxygène.

3. Procédé selon la revendication 1, caractérisé par le fait que l'on effectue la réaction à des températures de 200 à 300°C.

4. Procédé selon la revendication 1, caractérisé par le fait que l'on effectue la réaction sur un catalyseur contenant de l'oxyde de palladium en quantité de 0,5 à 10% en poids, avec 0,1 à 2% en poids de soufre et/ou de sélénium ou d'oxyde de sélénium.

5. Procédé selon la revendication 1, caractérisé par le fait que pour la préparation du p-cymène, on vaporise le limonène et on l'envoi sur le catalyseur chaud à l'aide d'azote qui sert de gaz véhicule.

## Claims

1. A process for preparing an alkylbenzene of the general formula I

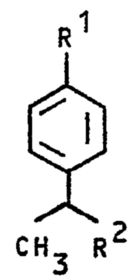

(I),

where $R^1$ is $C_1$–$C_4$-alkyl and $R^2$ is hydrogen or $C_1$–$C_4$-alky, by catalytic aromatization of an alkenylcyclohexene of the general formula II

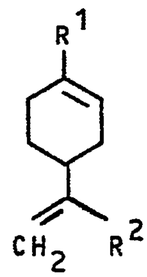

(II),

where $R^1$ and $R^2$ have the abovementioned meanings, which comprises carrying out the conversion in the gas phase at 150–350°C over a catalyst which contains palladium oxide and sulfur and/or selenium or selinium oxide on activated carbon.

2. A process as claimed in claim 1, wherein the conversion is carried out in the absence of air or oxygen.

3. A process as claimed in claim 1, wherein the conversion is carried out at 200–300°C.

4. A process as claimed in claim 1, wherein the conversion is carried out over a catalyst which contains palladium oxide in an amount of from 0.5 to 10% by weight and from 0.1 to 2% by weight of sulfur and/or selenium or selenium oxide.

5. A process as claimed in claim 1, wherein, to prepare p-cymene, limonene is vaporized and passed with nitrogen as carrier gas over the hot catalyst.